# EUROPEAN PATENT APPLICATION

(11) **EP 1 747 788 A1**
(43) Date of publication of application: **31.01.2007**
(21) Application number: 06117957.8
(22) Date of filing: 27.07.2006
(51) Int. Cl.: A61M 5/145, A61M 5/148, A61M 5/44

(54) **Device for conditioning liquids, particularly for biomedical use**

(30) Priority: 29.07.2005 IT MO20050196
(71) Applicant: Lean S.r.l., 41036 Medolla (MO) (IT)
(72) Inventor: Parrino, Andrea, 41037, Mirandola (MO) (IT)
(74) Representative: Modiano, Micaela Nadia

(57) **Abstract**

A device (1) for conditioning liquids, particularly for biomedical use, which comprises a hollow shell (2) and is provided internally with a receptacle (6) for at least one bag (S) for containing a liquid which is provided with an opening (P) for the outflow of the liquid, presser means (12) associated with the shell and adapted to compress the bag, and means (11) for maintaining the temperature of the liquid, which are associated with the shell (2) and are suitable alternatively to heat and cool the bag.

## Description

The present invention relates to a device for conditioning liquids, particularly for biomedical use.

It is known that in the biomedical field, liquids to be administered to patients (blood for transfusions, drip feeds, et cetera) are usually contained within appropriately sterilized bags which have, on their bottom, an opening for the outflow of the liquid and for its passage into the flexible tube of a biomedical line.

During administration operations, for example blood transfusions that must be performed during delicate surgical procedures, there is often the need to heat and/or cool the liquid contained in the biomedical line before it comes into contact with the patient.

It may also be necessary to administer the liquid more rapidly, i.e., it might be necessary to increase the flow-rate of liquid through the infusion tubes.

In order to heat the liquid to be administered, it is known to resort to particular heating systems, one of which is constituted by a cylinder which contains an electric resistor which keeps it warm; if necessary, the flexible tube of the biomedical line is wrapped around the warm cylinder by one of the assistants of the medical staff.

An alternative heating system entails inserting along the biomedical line a particular flat bag, which forms internally a coiled path which can be crossed by the liquid to be heated; the bag is accommodated between two mutually opposite electrical plates.

Moreover, the liquid to be administered is usually cooled by means of containers of frozen water, in which the infusion tube is immersed manually whenever the need is felt.

To increase the flow-rate of the liquid to be administered, one instead usually uses appropriate bag pressers, constituted by a sac or pouch provided with a chamber in which the bag that contains the liquid is inserted; the chamber, filled more or less gradually with air, increases in volume, compresses the bag and pushes the liquid contained therein so that it flows out more swiftly.

In some cases, the bag presser contains a built-in electric resistor, which allows to heat the liquid in the bag due to the Joule effect.

As an alternative to bag pressers, it is known to use pumps, generally of the positive-displacement type, which are inserted along the biomedical line and are intended to adjust the flow-rate of the liquid to a chosen value.

Traditional heating and cooling systems, as well as bag pressers and pumps of the known type, are not free from drawbacks, including the fact that whenever it is necessary to condition the liquid to be administered in terms of temperature and flow-rate, it becomes indispensable to combine the biomedical line with mutually separate and distinct devices, which often have a substantial overall volume.

The aim of the present invention is to eliminate the above mentioned drawbacks of the background art, by providing a device for conditioning liquids, particularly for biomedical use, which allows to adjust practically and easily the temperature and flow-rate of the liquid contained in a biomedical line.

Within this aim, an object of the present invention is to provide a device which has a simple and compact structure, has a limited space occupation, is relatively easy to provide in practice, safe in use and effective in operation, and also has a relatively low cost.

This aim and these and other objects, which will become better apparent hereinafter, are achieved by the present device for conditioning liquids, particularly for biomedical use, characterized in that it comprises a substantially hollow shell, which is provided internally with a receptacle for at least one bag for containing a liquid which is provided with at least one opening for the outflow of said liquid, and comprises heating means and presser means associated with said shell and respectively adapted to heat and compress said bag.

Further characteristics and advantages of the present invention will become better apparent from the following detailed description of two preferred but not exclusive embodiments of a device for conditioning liquids, particularly for biomedical use, illustrated by way of non-limiting example in the accompanying drawings, wherein:
Figure 1 is a side elevation view of a first embodiment of the device according to the invention, mounted on a vertical supporting post;
Figure 2 is a sectional view, taken along a substantially vertical plane, of the device of Figure 1 prior to the activation of the presser means;
Figure 3 is a sectional view, taken along a substantially vertical plane, of the device of Figure 1 during the activation of the presser means;
Figure 4 is a sectional view, taken along a substantially horizontal plane, of the device of Figure 1;
Figure 5 is a sectional view, taken along a substantially vertical plane, of a second embodiment of the device according to the invention.

With reference to the figures, the reference numeral 1 generally designates a device for conditioning liquids, particularly for biomedical use.

The device 1 can be applied usefully to a vertical supporting post A of the type of drip-feed supporting posts, known as stands, normally commonly used in hospitals and clinics.

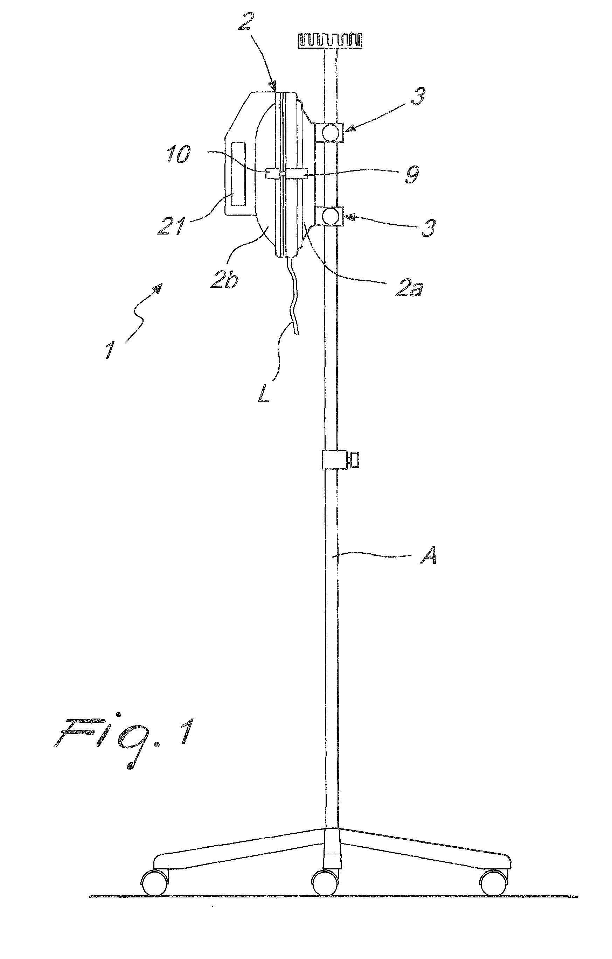

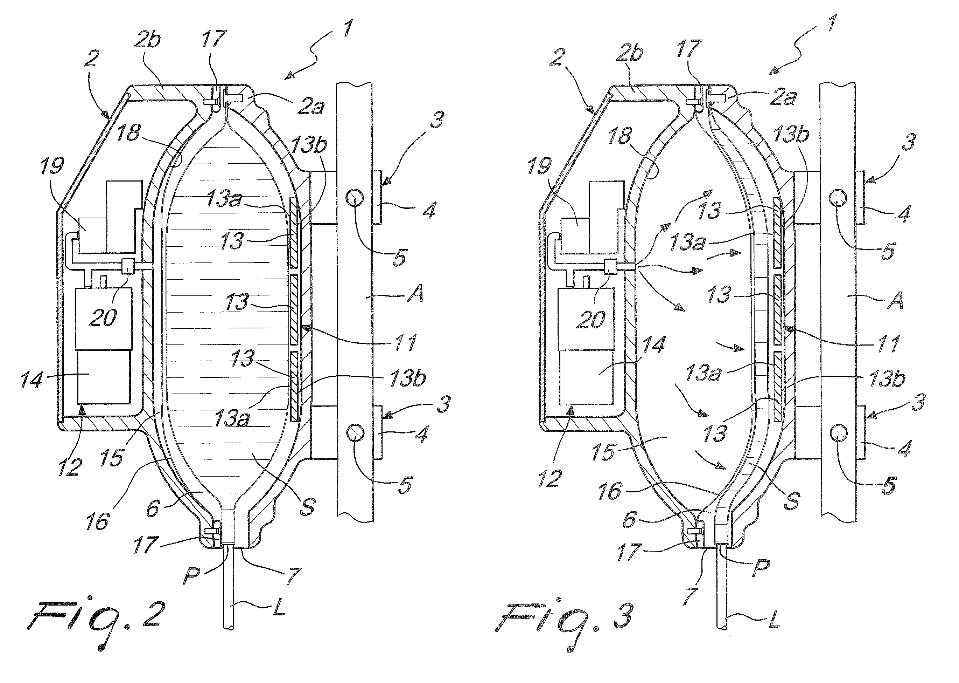

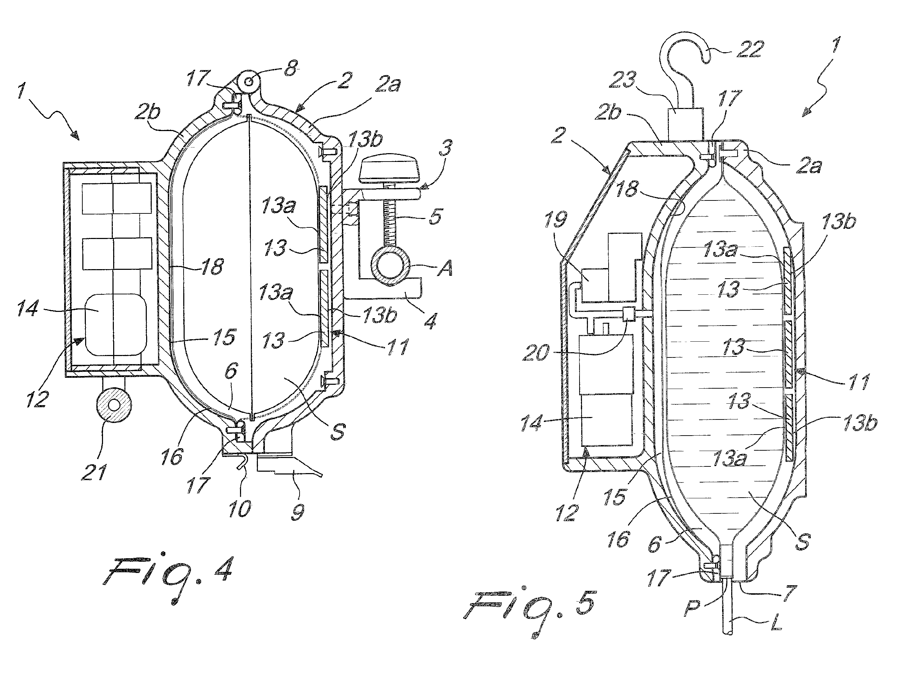

In a first embodiment, shown in Figures 1 to 4, the device 1 in particular comprises a hollow shell 2, which can be associated with the post A by virtue of fixing means 3 of the friction type, which comprise two supports 4 which protrude monolithically with respect to the shell 2 and can be positioned around the post A, and two locking screws 5, which are received so as to pass through one side of the supports 4 and can be screwed against the post A.

By way of the rotation of the locking screws 5 it is possible in practice to lock the post A in abutment against the supports 4, rigidly coupling the shell 2 to said post, or to disengage said post from the supports 4, so as to allow the removal or transfer of the device 1.

The shell 2 is provided internally with a receptacle 6 for a bag S for containing a liquid for biomedical use, such as drip-feeds, blood or other physiological fluids.

The bag S is provided with an opening P for the outflow of the liquid and the passage of said liquid into a biomedical line L for administration to a patient.

Once it has been placed in the receptacle 6, the bag S is kept substantially vertical and its opening P is arranged downward at a passage port 7, which is formed in the shell 2 and allows to connect it to the biomedical line L.

The shell 2 is constituted by a first half-shell 2a and a second half-shell 2b, which are mutually associated at an adjacent side by way of the interposition of rotation means 8 of the hinge type, which are suitable to articulate them so that they can oscillate between a mutually closed configuration, in which the receptacle 6 is substantially separated from the outside, and a mutually open configuration, in which the receptacle 6 can be accessed from outside to allow the positioning and replacement of the bag S within the shell 2.

In the mutual closure configuration, the half-shells 2a and 2b can be joined monolithically by way of removable locking means constituted for example by a movable tab 9, which is associated with the first half-shell 2a on the opposite side with respect to the rotation means 8 and can engage a bracket 10, which is formed monolithically on the second half-shell 2b, in order to retain close to each other the free sides of the half-shells 2a and 2b.

The device 1 according to the invention is provided, within the shell 2, with means 11 for maintaining the temperature of the liquid which are suitable to alternately heat and cool the bag S and the liquid inside it, and with presser means 12 adapted to compress the bag S in order to force the outflow of the liquid through the opening P.

The temperature holding means 11 comprise a plurality of Peltier cells 13, which are provided with a first face 13a and a second face 13b adapted to exchange heat between them; the cells are suitable to heat and cool the liquid contained in the bag S.

The Peltier cells 13 are arranged within the first half-shell 2a with the first faces 13a directed toward the inside of the receptacle 6.

Once it has been fitted within the shell 2, the bag S is arranged proximate to the first faces 13a of the Peltier cells 13 and exchanges heat with them.

In an alternative embodiment of the invention, the temperature holding means 11 can be provided with separate thermal actuators for heating and cooling the liquid contained in the bag S. In this case, for example, the thermal actuators for heating can provide one or more heating elements with an electric resistor of appropriate heating power arranged within the shell 2 with a respective heat exchange surface that faces the bag S. The thermal actuators for cooling can instead have at least one refrigeration module of suitable heating power arranged within the shell 2 and provided with two heat exchange surfaces, one of which faces the receptacle 6 in order to remove heat from the bag S.

Advantageously, the presser means 12 comprise a pump 14 for sending a working fluid, preferably air, into a hermetic chamber 15, which is provided in the second half-shell 2b and is separated from the receptacle 6 by interposing a flexible membrane 16, proximate to which the bag S can be positioned.

In particular, the edge of the membrane 16 is kept in contact with the internal surface of the second half-shell 2b by means of a locking collar 17 screwed to said half-shell.

In practice, the hermetic chamber 15 is formed by the space between the internal wall 18 of the second half-shell 2b and the membrane 16; in the configuration in which the half-shells 2a and 2b are mutually closed, the receptacle 6 is instead formed by the space between the Peltier cells 13 and the membrane 16, which face each other.

The hermetic chamber 15 can be replaced equivalently by an air-filled chamber located between the internal wall of the shell 2 and the bag S.

The presser means 12 further comprise venting means which are interposed between the hermetic chamber 15 and the outside, which allow, when necessary, the outflow of the working fluid from the hermetic chamber 15.

Said venting means are constituted for example by an electric valve 19, which is accommodated together with the pump 14 in a compartment of the second half-shell 2b which is formed adjacent to the internal wall 18, on the opposite side with respect to the hermetic chamber 15.

The electric valve 19 is connected to the pneumatic circuit for connecting the pump 14 to the hermetic chamber 15.

The operation of the temperature holding means 11 and of the presser means 12 can be controlled by means of an electronic management system, not shown in the figures, such as a preferably programmable electronic board which controls the switching on and off of the Peltier cells 13 and of the pump 14 as well as the opening and closure of the electric valve 19.

In order to perform all of these functions, the device 1 is provided with means for supplying electric power to the user devices, both of the temperature holding means 11 and of the presser means 12.

Said power supply means, not shown in detail in the figures, are constituted preferably by one or more rechargeable batteries and/or by a power supply, which can be connected to the ordinary electrical mains and is suitable to recharge said batteries or to power the user devices directly.

Conveniently, the presser means 12 are provided with suitable means for detecting the pressure of the liquid contained in the bag S, which are constituted for example by an electric pressure transducer 20, which is interposed along the pneumatic circuit for connecting the pump 14 to the hermetic chamber 15.

The electric pressure transducer 20 in practice allows to measure the pressure of the air sent to the hermetic chamber 15 by the pump 14 and to supply a corresponding electrical signal to the electronic management system, which allows to control the pressure of the liquid in the bag S; the higher the pressure of the liquid within the bag S, the higher the flow-rate that characterizes its outflow and its introduction in the biomedical line L.

The shell 2 has particularly compact dimensions which are consistent with those of ordinary commercially available bags S; once it has been disengaged from the post A, the device 1 can be gripped and carried by hand by virtue of particular grip means 21, constituted for example by a handle associated with the second half-shell 2b.

In an alternative embodiment of the invention, shown in Figure 5, the device 1 is suitable to be hung for example from the post A by virtue of hanging means such as a hook 22 associated with one of the half-shells 2a or 2b. Advantageously, between the shell 2 and the hook 22 there are weighing means, such as a conventional load cell 23 functionally connected in input to the electronic management system. The load cell 23 is activated periodically by the electronic management system and the acquired data are then processed by said system in order to calculate the flow-rate of liquid that flows out from the bag S and/or the total quantity of liquid that has flowed out.

Conveniently, it is possible to set in the electronic management system a nominal flow-rate value, so that if the flow-rate value that is calculated periodically by said system is lower than the set nominal value, the presser means 12 are activated in order to increase the flow of liquid in output from the bag S.

In this manner, therefore, a feedback control is performed which allows the electronic management system to affect actively the presser means 12 in order to optimize the operation of the device 1.

It is also possible to set in the electronic management system the total quantity of liquid to be administered to the patient, so that the electronic management system updates periodically, according to the values progressively detected by the load cell 23, the quantity that has already been dispensed and optionally activates indicator means of the visual and/or acoustic type, not shown, when the set quantity to be administered is approached or reached.

Finally, it is possible to set in the electronic management system a minimum flow-rate value in output from the bag S, so that said system periodically compares the calculated flow-rate value with the minimum set value and activates any additional indicator means of the visual and/or acoustic type if the detected value is equal to, or lower than, the minimum set value.

When treatment begins, the operator inserts the bag S full of liquid in the receptacle 6 and activates the device 1. The electronic management system resets the weight detected by the load cell 23 and operates, at preset intervals, said cell in order to detect the current weight and therefore the difference in weight with respect to the instant when the operation of the device 1 was started and/or with respect to the previous sampling time, so as to calculate respectively the quantity of liquid that has flowed out of the bag S and/or its current flow-rate, and can optionally activate interventions to correct the flow by acting on the presser means 12.

The electronic management system, on the basis of the set data (the nominal flow-rate value and optionally the quantity of liquid to be administered and the minimum flow-rate in output from the bag S), every time the weight is detected by the cell 23, performs the operations described above, actuating the presser means and activating the indicator means and the additional indicator means.

Conveniently, it is noted that the device 1 can be positioned proximate to the patient without necessarily being arranged in an elevated position, as ordinarily occurs for known types of gravity-based device. The correct delivery of liquid by the device 1 is in fact ensured by the action of the presser means 12 instead of being based on the difference in head between the height of the bag and the height of the delivery point.

This fact therefore allows to use the device 1 even in situations in which it can be difficult or otherwise awkward to position the bag S in an elevated position.

In practice it has been found that the described invention achieves the proposed aim and objects, and in particular the fact is stressed that by means of a compact structure which has considerably limited space occupation it allows to condition easily the temperature and flow-rate of the liquids contained in ordinary bags used in the biomedical field.

The invention thus conceived is susceptible of numerous modifications and variations, all of which are within the scope of the appended claims.

All the details may further be replaced with other technically equivalent elements.

In practice, the materials used, as well as the contingent shapes and dimensions, may be any according to requirements without thereby abandoning the scope of the protection of the appended claims.

The disclosures in Italian Patent Application No. MO2005A000196, from which this application claims priority, are incorporated herein by reference.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A device for conditioning liquids, particularly for biomedical use, **characterized in that** it comprises a substantially hollow shell, which is provided internally with a receptacle for at least one bag for containing a liquid which is provided with at least one opening for the outflow of said liquid, presser means associated with said shell and adapted to compress said bag, and means for maintaining the temperature of said liquid, which are associated with said shell and are suitable to alternatively heat and cool said bag.

2. The device according to claim 1, **characterized in that** said shell comprises at least one first half-shell and one second-half shell, which are mutually associated by interposing rotation means which are suitable to articulate them so that they can oscillate between a mutually closed configuration, in which said receptacle is substantially isolated from the outside, and a mutually open configuration, in which said receptacle can be accessed from the outside.

3. The device according to one or more of the preceding claims, **characterized in that** said temperature holding means comprise at least one Peltier cell, which is arranged within said shell and is provided with a first face and a second face which are adapted to exchange heat with each other, said first face facing said receptacle in order to exchange heat with said bag.

4. The device according to one or more of the preceding claims, **characterized in that** said temperature holding means comprise a plurality of said Peltier cells.

5. The device according to one or more of claims 1 and 2, **characterized in that** said temperature holding means comprise separate thermal actuators for heating and cooling said liquid which are associated with said shell and are adapted to cooperate with said bag.

6. The device according to claim 5, **characterized in that** said thermal actuators for heating said liquid comprise at least one heating element of the electrical resistor type.

7. The device according to claim 5, **characterized in that** said thermal actuators for cooling said liquid comprise at least one refrigeration module.

8. The device according to one or more of the preceding claims, **characterized in that** said presser means comprise at least one pump for sending a working fluid into a hermetic chamber, which is formed within said shell and is separated from said receptacle by way of the interposition of a flexible membrane, proximate to which said bag can be positioned.

9. The device according to one or more of the preceding claims, **characterized in that** said working fluid is air.

10. The device according to one or more of the preceding claims, **characterized in that** said presser means comprise means for venting said working fluid from said hermetic chamber.

11. The device according to one or more of the preceding claims, **characterized in that** said venting means comprise at least one electric valve, which is interposed between said hermetic chamber and the outside.

12. The device according to one or more of the preceding claims, **characterized in that** said presser means comprise means for detecting the pressure of said working fluid.

13. The device according to one or more of the preceding claims, **characterized in that** said detection means comprise at least one electrical pressure transducer which is interposed substantially between said pump and said hermetic chamber.

14. The device according to one or more of the preceding claims, **characterized in that** said hermetic chamber is formed by the space comprised between said membrane and the inside wall of said shell which face each other.

15. The device according to one or more of the preceding claims, **characterized in that** said receptacle is substantially formed by the space comprised between said Peltier cells and said membrane which face each other.

16. The device according to one or more of claims 1 to 7, **characterized in that** said presser means comprise at least one pump for sending a working fluid into an air-filled chamber which is accommodated within said shell and is adapted to form said receptacle.

17. The device according to one or more of the preceding claims, **characterized in that** it comprises means for supplying electric power to at least one between said temperature holding means and said presser means.

18. The device according to one or more of the preceding claims, **characterized in that** said electric power supply means comprise at least one battery.

19. The device according to one or more of the preceding claims, **characterized in that** said electric power supply means comprise at least one power supply which can be associated with the ordinary electrical mains.

20. The device according to one or more of the preceding claims, **characterized in that** it comprises means for gripping said shell.

21. The device according to one or more of the preceding claims, **characterized in that** said grip means comprise at least one handle.

22. The device according to one or more of the preceding claims, **characterized in that** it comprises an electronic system for the management of said presser means and of said temperature holding means.

23. The device according to claim 22, **characterized in that** said electronic management system comprises a programmable electronic board.

24. The device according to one or more of the preceding claims, **characterized in that** it comprises means for fixing said shell to a substantially vertical supporting rod.

25. The device according to one or more of the preceding claims, **characterized in that** said fixing means are of the friction type.

26. The device according to one or more of the preceding claims, **characterized in that** said fixing means comprise at least one support, which is associated with said shell and can be positioned around said post, and at least one locking screw, which passes through said support and can be screwed against said post.

27. The device according to one or more of claims 1 to 23, **characterized in that** it comprises hanger means which can be associated with a supporting post or the like.

28. The device according to claim 27, **characterized in that** said hanger means comprise at least one hook.

29. The device according to one or more of the preceding claims, **characterized in that** it comprises weighing means, which are interposed between said hanger means and said shell and can be functionally connected in input to said electronic management system, said system being adapted to receive periodic sensings of the weighing means in order to calculate the flow-rate of liquid in output from the bag and/or the total quantity that has flowed out of said bag.

30. The device according to claim 29, **characterized in that** said weighing means comprise at least one load cell associated with at least one of said half-shells.

31. The device according to one or more of the preceding claims, **characterized in that** said electronic management system is adapted to compare the current flow-rate value with a nominal flow-rate value that has been set and to activate said presser means if the current flow-rate value is lower than the nominal flow-rate value.

32. The device according to one or more of the preceding claims, **characterized in that** it comprises indicator means which are activated by said electronic management system when a set value of total quantity of liquid to be administered is approached or reached.

33. The device according to one or more of the preceding claims, **characterized in that** said electronic management system is adapted to compare the current flow-rate value with a set minimum flow-rate value and to activate additional indicator means if the current flow-rate value is lower than, or equal to, the minimum one.
